(19) Europäisches Patentamt
European Patent Office
Office européen des brevets

(11) Veröffentlichungsnummer: **0 000 482**
A1

(12) # EUROPÄISCHE PATENTANMELDUNG

(21) Anmeldenummer: 78100289.4

(22) Anmeldetag: 30.06.78

(51) Int. Cl.[2]: **C 07 D 493/04**, C 07 D 307/79
//C07D307/83,C07C49/82,
(C07D493/04,311/00,307/00)

(30) Priorität: **29.07.77 US 820265**
**25.05.78 GB 22501/78**

(43) Veröffentlichungstag der Anmeldung:
**07.02.79 Bulletin 79/3**

(84) Benannte Vertragsstaaten:
BE CH DE FR GB NL

(71) Anmelder: **F.Hoffmann-La Roche & Co.**
**Aktiengesellschaft Abt. VIII-Pt**
**CH-4002 Basel. (CH)**

(72) Erfinder: **Liebman, Arnold A.**
**7 Belleclaire Place Verona,**
**N.J.. (US)**

(72) Erfinder: **Liu, Yu-Ying**
**8 Passmore Street Westwood,**
**N.J.. (US)**

(74) Vertreter: **Mahé, Jean et al**
**Postfach 601 Grenzacherstrasse 124**
**CH-4002 Basel. (CH)**

(54) **Verfahren zur Herstellung von 8-Methoxy-psoralen und Zwischenprodukte in dieser Herstellung.**

(57) Herstellung des 8-Methoxypsoralens (I) aus 6-Hydroxy-7-methoxy-3 (2H)-benzofuranon nach folgendem Schema

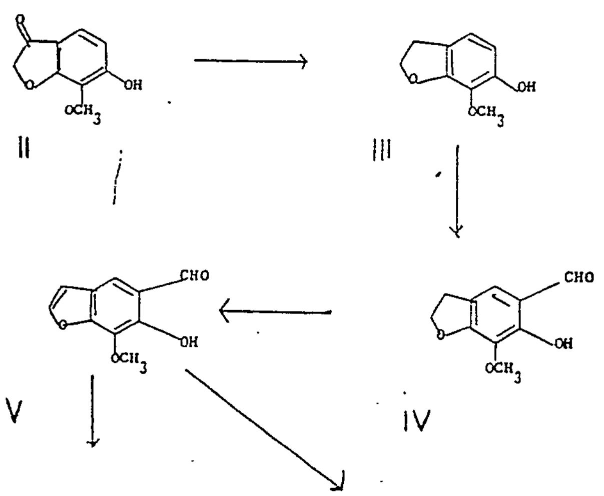

II III IV V

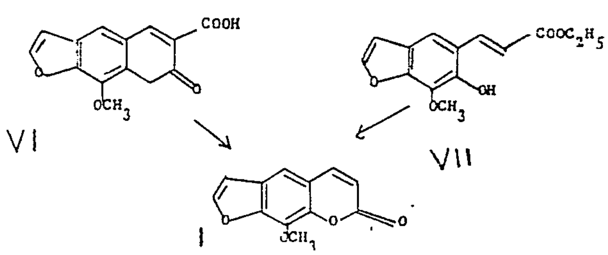

VI VII I

EP 0 000 482 A1

Croydon Printing Company Ltd.

RAN 4051/4

F. Hoffmann-La Roche & Co. Aktiengesellschaft, Basel/Schweiz

Verfahren zur Herstellung von 8-Methoxy-psoralen und Zwischenprodukte in dieser Herstellung.

Die Erfindung betrifft ein neues Verfahren zur Herstellung des bekannten pharmakologisch aktiven 8-Methoxypsoralens, einer Verbindung der Formel

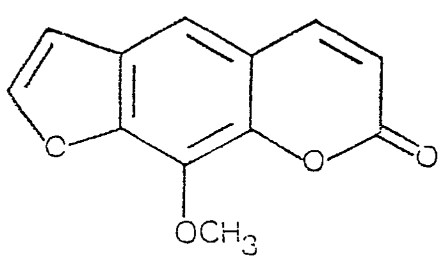

sowie neue in diesem Verfahren verwendete Zwischenprodukte.

Das Verfahren besteht darin, dass man (A) eine Verbindung

Mé/12.6.1978

der Formel

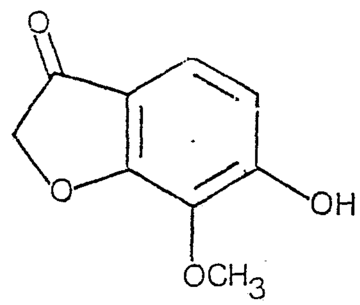

II

katalytisch hydriert,

(B) das Produkt von (A) mit einem Formylierungsmittel umsetzt,

(C) das Produkt von (B) mit einem Dehydrierungsmittel umsetzt und entweder

(D) das Produkt von (C) mit Malonsäure oder einem funktionellen Derivat davon umsetzt und

(E) das Produkt von (D) decarboxyliert, oder

(F) das Produkt von (C) der Formel

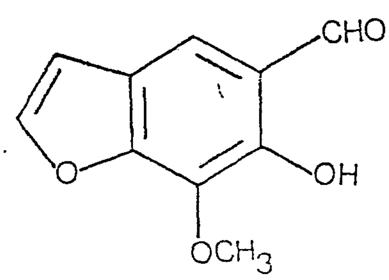

V

durch Wittig-Kondensation mit einem Phosphoranderivat in ein Gemisch des Endproduktes und einer Verbindung der Formel

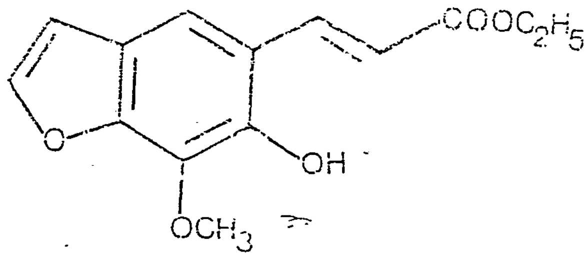

VII

überführt und

(G) die Verbindung der Formel VII zum Endprodukt cyclisiert.

Im nachstehenden Reaktionsschema sind die verschiedenen Stufen des obigen Verfahrens sowie die neuen Zwischenprodukte der Formeln III, V, VI und VII dargestellt.

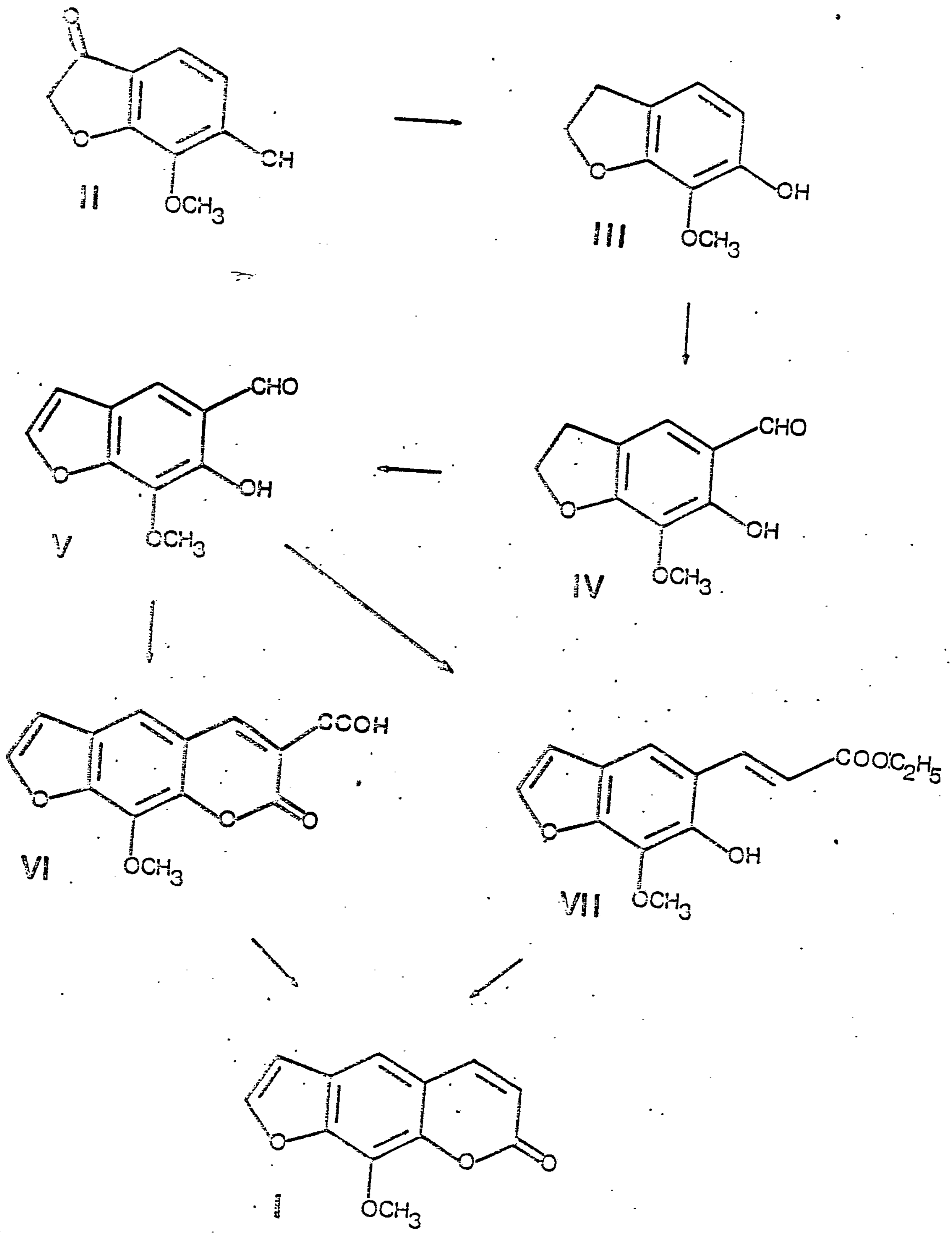

II

III

IV

V

VI

VII

I

Stufe A (II → III)

Das Ausgangs[illegible] der Formel II, welches wie in J.A.C.S. [illegible] beschrieben ausgehend von 2,6-Dihydroxyanisol hergestellt werden kann, wird z.B. unter Verwendung eines Edelmetallkatalysators wie Palladium/Kohlenstoff oder Platin, in einem Lösungsmittel wie Essigsäure, bei einer Temperatur zwischen etwa 15 und 50°C und unter normalem Druck oder unter einem Druck von bis zu 10 Atmosphären, wobei Raumtemperatur und ein Druck von 3 Atmosphären bevorzugt sind, katalytisch hydriert.

Stufe B (III → IV)

Die Verbindung der Formel III wird mit einem Formylierungsmittel, z.B. Zinkcyanid und Salzsäure (Gattermann-Reaktion), zweckmässigerweise bei Raumtemperatur umgesetzt.

Stufe C (IV → V)

Die Verbindung der Formel IV wird z.B. mit Dichlordicyanbenzochinon in einem inerten Lösungsmittel, wie Dioxan, einem cyclischen Aether, z.B. Tetrahydrofuran, oder Benzol, bei einer Temperatur von der Rückflusstemperatur des Reaktionsgemisches bis zu Raumtemperatur, wobei letztere bevorzugt ist, dehydriert.

Stufe D (V → VI)

Die Verbindung der Formel V wird mit Malonsäure oder einem funktionellen Derivat davon, z.B. Diäthylmalonat oder vorzugsweise Aethylcyanacetat, umgesetzt. Die Reaktion mit letzterem wird vorzugsweise in einem polaren Lösungsmittel, z.B. Wasser, und bei Raumtemperatur durchgeführt.

Stufe E (VI → I)

Die Verbindung der Formel VI wird zweckmässigerweise durch Erhitzen, z.B. oberhalb von [illegible]°C, in Gegenwart von Kalciumcarbonat decarboxyliert.

BAD ORIGINAL

Stufe F (V ⟶ VII + I)

Die Verbindung der Formel V wird mit einem Phosphoranderivat, vorzugsweise $(C_6H_5)_3$ P = $CH\text{-}COOC_2H_5$, zweckmässigerweise in einem polaren oder nicht polaren inerten organischen Lösungsmittel, wie Methanol, Tetrahydrofuran, Dioxan, einem aromatischen Kohlenwasserstoff , z.B. Benzol, einem hochsiedenden Aether oder Dimethylsulfoxyd, bei einer Temperatur von Raumtemperatur bis zu Rückflusstemperatur des Reaktionsgemisches, wobei letztere Temperatur bevorzugt ist, in einer Wittig-Kondensation umgesetzt.

Stufe G (VII ⟶ I

Die Verbindung der Formel VII wird in an sich bekannter Weise durch Erhitzen, zweckmässigerweise unter Stickstoff, bei einer Temperatur von etwa 200°C, während einer Zeitdauer von mindestens 30 Stunden, oder durch Bestrahlen cyclisiert.

00034

## Beispiel

Eine Lösung von 6-Hydroxy-7-methoxy-3(2H)-benzo-furanon (233 mg, 1.29 mmol) in 5 ml Essigsäure wird 4 Stunden bei Raumtemperatur in Gegenwart von 50 mg 10% Pd/C hydriert. Das Gemisch wird filtriert und unter Vakuum eingeengt. Der Rückstand wird auf Silicagel chromatographiert und mit $CHCl_3$ eluiert. Nach Eindampfen erhält man 2,3-Dihydro-7-methoxy-6-benzofuranol als farbloses Oel.

Ein Gemisch von 2.49 g (15 mMol) 2,3-Dihydro-7-methoxy-6-benzofuranol, 5.87 g (50 mMol) Zinkcyanid und 100 ml trockenem Aether wird eine Stunde bei 0°C, dann 2 Stunden bei Raumtemperatur mit Chlorwasserstoff gesättigt. Das Gemisch wird über Nacht bei Raumtemperatur gelassen, dann unter Vakuum eingeengt und der Rückstand eine Stunde unter Rückfluss mit 100 ml 0.1N HCl behandelt. Das Gemisch wird gekühlt und mit Aether extrahiert. Das Produkt wird über Silicagel mit $CHCl_3$ als Eluierungsmittel chromatographiert. Man erhält 2,3-Dihydro-6-hydroxy-7-methoxy-5-benzofurancarboxaldehyd als farblosen Feststoff vom Schmelzpunkt 71-72°C.

Eine Lösung von 817 ml (3.6 mMol) DDQ in 10 ml Dioxan wird 582 mg (3 mMol) 2,3-Dihydro-6-hydroxy-7-methoxy-5-benzofurancarboxaldehyd in 15 ml Dioxan zugesetzt, das Gemisch 7 Stunden unter Rückfluss erhitzt, abgekühlt und der entstandene Feststoff abfiltriert. Der Niederschlag wird zuerst mit Benzol, dann mit $CHCl_3$ gewaschen. Die mit dem ursprünglichen Filtrat vereinigten Waschwässer werden unter Vakuum eingeengt. Der Rückstand wird zuerst auf 10 trockenem Silicagel mit $CHCl_3$ als Eluierungsmittel chromatographiert, dann durch Chromatographie über 20 g Siliziumoxyd in Benzol-Aethylacetat (95:5) mit letzterem Lösungsmittelgemisch als Eluierungsmittel chromatographiert. Man erhält 6-Hydroxy-7-methoxy-5-benzofurancarboxaldehyd als gelben Feststoff von Schmelzpunkt 60-62°C.

BAD ORIGINAL

Hydroxy-7-methoxy-5-benzofurancarboxaldehyd (192 mg, 1 mMol) in 1 ml Wasser wird einer Lösung von Aethylcyanacetat (1?? mg, 1.1 mMol) in 1 ml 200 mg Natriumhydroxyd enthaltendem Wasser zugesetzt. Das Gemisch wird 20 Stunden bei Zimmertemperatur gerührt und 30 Minuten mit 6 ml 2N HCl unter Rückfluss behandelt. Nach Abkühlen wird das Produkt durch Zentrifugieren und Kristallisation aus Aethanol isoliert. Man erhält 7-oxo-9-Methoxy-7H-furo-[3,2-g][1]benzopyran-6-carbonsäure als gelben Feststoff vom Schmelzpunkt 227-228°C.

Ein Gemisch von 6-Hydroxy-7-methoxy-5-benzofurancarboxaldehyd (192 mg, 1 mMol) und 666 mg (1.9 mMol) Carbäthoxymethylentriphenylphosphoran (welches wie in J.Org.Chem., 27, 998, 1962 beschrieben aus Triphenylphosphin und Aethylbromacetat erhalten wurde) in 20 ml Benzol wird 2 Stunden unter Rückfluss erhitzt. Das Lösungsmittel wird durch Destillation unter Vakuum abgedampft und der Rückstand über Silicagel mit Benzol chromatographiert. Man erhält 6-Hydroxy-7-methoxybenzofuran-5-trans-acrylsäureäthylester als farblosen Feststoff vom Schmelzpunkt 127-128°C.

2?? mg (0.923 mMol) 7-oxo-9-Methoxy-7H-furo[3,2-g][1]benzopyran-6-carbonsäure wird mit 300 mg $CaCO_3$ vermischt und das Gemisch 45 Minuten unter Stickstoff bei 10?°C erhitzt. Nach Abkühlen wird das Gemisch mit $CHCl_3$ vermischt und filtriert und das Filtrat unter Vakuum eingeengt. Der Rückstand wird über Silicagel mit Benzol als Elulerungsmittel chromatographiert. Man erhält einen farblosen Feststoff vom Schmelzpunkt 146-148°C, welcher sich durch Dünnschichtchromatographie mit 5% Aethylacetat in Benzol als ... mittel als identisch mit authentischem 9-Methoxyfuro-... [3,2-g][1]benzopyran-7-on (8-Methoxypsoralen oder Methoxsalen) erwies.

... Mol) ... Hydroxy-7-methoxybenzofuran ... unter Stickstoff auf ... und ?? Stunden bei dieser T... erhalten ... wird in Benzol ... gelöst ...

Benzol als Eluierungsmittel [illegible]. Auch das [illegible] Produkt erwies sich als identisch mit authentischem [illegible].

Das Ausgangsmaterial kann wie folgt hergestellt werden:

Das, wie in JACS 73, 5765, 1951 beschrieben, aus 19.6 g (0.14 Mol) 2,6-Dihydroxyanisol, 10.7 g (0.14 Mol) Chloracetonitril und 10 g frisch geschmolzenem Zinkchlorid erhaltene rohe Produkt wird durch Chromatographie auf Silicagel mit Chloroform als Eluierungsmittel und Kristallisation aus Wasser-Methanol gereinigt. Man erhält α-Chlor-2,4-dihydroxy-3-methoxyacetophenon als farblosen Feststoff vom Schmelzpunkt 71-72°C.

Wie in dem oben erwähnten Artikel beschrieben werden 23.4 g (0.108 Mol) α-Chlor-2,4-dihydroxy-3-methoxyacetophenon mit einer Lösung von 29 g wasserfreiem Natriumacetat in 80 ml absolutem Aethanol behandelt. Nach Umkristallisieren aus Methanol erhält man 6-Hydroxy-7-methoxy-3(2H)-benzofuranon als farbloser Feststoff vom Schmelzpunkt 160°C.

BAD ORIGINAL

Patentansprüche

1. Verfahren zur Herstellung einer Verbindung der Formel

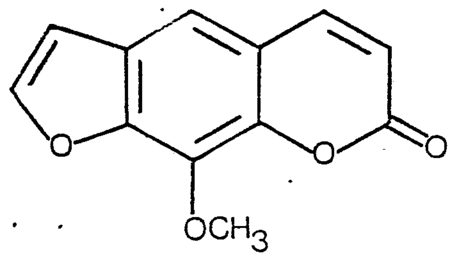

I

dadurch gekennzeichnet, dass man (A) eine Verbindung der Formel

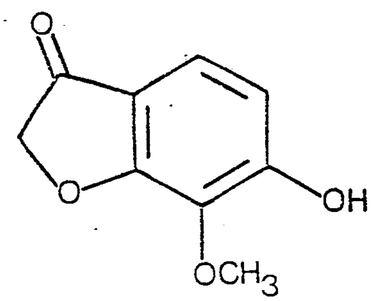

II

katalytisch hydriert

(B) das Produkt von (A) mit einem Formylierungsmittel umsetzt,

(C) das Produkt von (B) mit einem Dehydrierungsmittel behandelt und entweder

(D) das Produkt von (C) mit Malonsäure oder einem funktionellen Derivat davon umsetzt und

(E) das Produkt von (D) decarboxyliert oder

(F) das Produkt von (C) der Formel

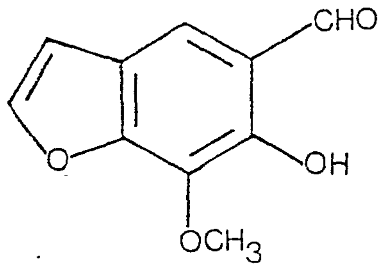

V

durch Wittig-Kondensation mit einem Phosphoranderivat zu einem Gemisch des Endproduktes und einer Verbindung der Formel

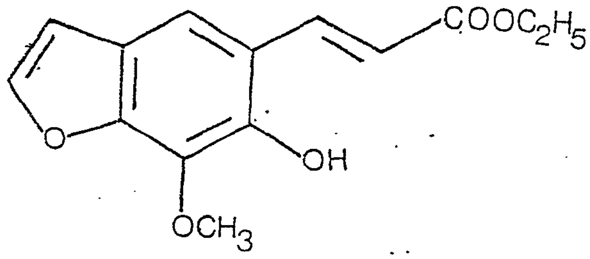

VII

umsetzt und

(G) die Verbindung der Formel VII durch Erhitzen oder Bestrahlung zum Endprodukt cyclisiert.

2. Verfahren nach Anspruch 1, dadurch gekennzeichnet, dass die Hydrierungsstufe (A) mit einem Edelmetall in Essigsäure, die Formylierungsstufe (B) mit Zinkcyanid und Salzsäure und die Dehydrierungsstufe (C) mit Dichlordicyanobenzochinon in einem inerten Lösungsmittel durchgeführt werden, das Produkt von Stufe (C) mit Aethylcyanacetat in einem polaren Lösungsmittel oder mit dem Phosphoran $(C_6H_5)_3P=CH-COOC_2H_5$ umgesetzt und die Verbindung der Formel VII 30 Stunden bei einer Temperatur von etwa 200°C erhitzt wird.

BAD ORIGINAL

0000482

7. Eine Verbindung der Formel

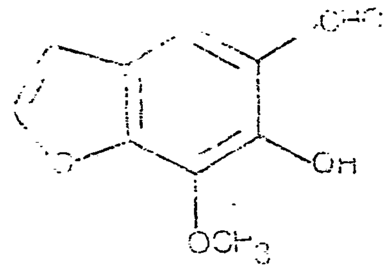

V

Eine Verbindung der Formel

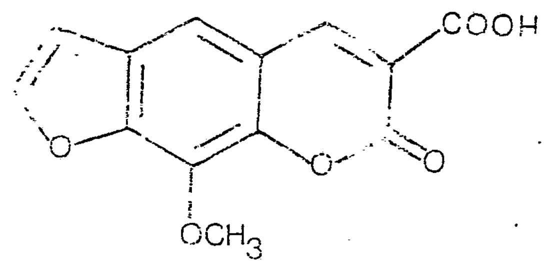

VI

Eine Verbindung der Formel

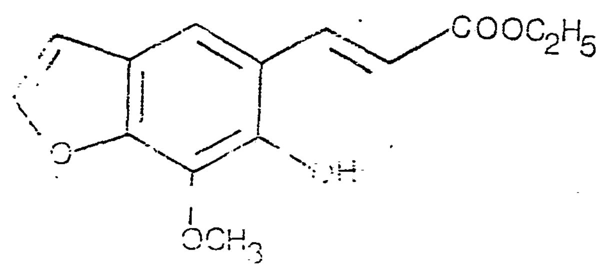

VII

BAD ORIGINAL

 0000432

6. Eine Verbindung der Formel

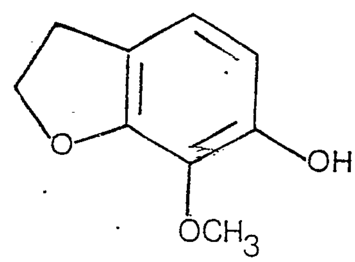

III

***

Europäisches Patentamt

# EUROPÄISCHER RECHERCHENBERICHT

0000482

Nummer der Anmeldung
EP 78 10 0289

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | CHEMICAL ABSTRACTS 75, 151709n(1971)<br>* Zusammenfassung *<br>-- | 1 |
| | JOURNAL OF ORGANIC CHEMISTRY, 34, 2311-13 (1969)<br>* Seite 2313, Spalte 2, Zeilen 8-40 *<br>-- | 1 |
| | CHIMICA THERAPEUTICA, 9, 335-40(1974)<br>* Seite 337, Spalte 1, Zeilen 41-50; Seite 338, Spalte 1, Zeilen 1-7 *<br>-- | 1 |
| A | JOURNAL OF THE AMERICAN CHEMICAL SOCIETY 72, 4826-29 (1950)<br>* Seite 4827, Spalte 2, Zeilen 50-75; Seite 4828, Spalte 1, Zeilen 1-8 *<br>---- | 5 |

KLASSIFIKATION DER ANMELDUNG (Int.Cl.²)

C 07 D 493/04
C 07 D 307/79
//C 07 D 307/83
C 07 C 49/82
(C 07 D 493/04
C 07 D 311/00
C 07 D 307/00)

RECHERCHIERTE SACHGEBIETE (Int. Cl.²)

C 07 D 493/04
C 07 D 307/79//
(C 07 D 493/04
C 07 D 311/00
C 07 D 307/00)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 26-09-1978 | ALFARO |